Europäisches Patentamt

European Patent Office

Office européen des brevets

(19)

(11) Veröffentlichungsnummer : **0 025 514**
**B1**

(12) # EUROPÄISCHE PATENTSCHRIFT

(45) Veröffentlichungstag der Patentschrift :
16.02.83

(21) Anmeldenummer : **80104795.2**

(22) Anmeldetag : **13.08.80**

(51) Int. Cl.³ : **C 12 N 9/18// C12Q1/60,
C12R1/38**

(54) **Verfahren zur Gewinnung von Cholesterinesterase.**

(30) Priorität : **20.08.79 DE 2933648**

(43) Veröffentlichungstag der Anmeldung :
**25.03.81 Patentblatt 81/12**

(45) Bekanntmachung des Hinweises auf die Patenterteilung : **16.02.83 Patentblatt 83/07**

(84) Benannte Vertragsstaaten :
**AT BE CH DE FR GB IT LI LU NL SE**

(56) Entgegenhaltungen :
**DE A 2 527 068**

**CHEMICAL ABSTRACTS, Band 86, Nr. 19, 9. Mai
1977, Seite 403, Zusammenfassung Nr. 137972 p,
Columbus, Ohio, US
AGRICULT. BIOL. CHEM., Band 40, Nr. 8 1976, T.
UWAJIMA et al. : « Production of cholesterol
esterase by Pseudomonas fluorescens », Seiten
1605-1609
CHEMICAL ABSTRACTS, Band 92, Nr. 3, 21.
Januar 1980, Seite 530, Zusammenfassung Nr.
20576v, Columbus, Ohio, US**

(73) Patentinhaber : **BOEHRINGER MANNHEIM GMBH
Sandhofer Strasse 112-132 Postfach 31 01 20
D-6800 Mannheim 31-Waldhof (DE)**

(72) Erfinder : **Beaucamp, Klaus, Dr.
Von-Kühlmann-Strasse 15
D-8132 Tutzing (DE)**
Erfinder : **Nelboeck, Michael, Dr.
Bareislweg 21
D-8132 Tutzing (DE)**
Erfinder : **Gauhl, Helmgard
Kustermannstrasse 31
D-8132 Tutzing (DE)**
Erfinder : **Seidel, Hans, Dr.
Waxensteinstrasse 6
D-8132 Tutzing (DE)**
Erfinder : **Gruber, Wolfgang, Dr.
Am Oberanger 7
D-8132 Tutzing-Unterzeismering (DE)**
Erfinder : **Brunner, Herwig, Dr.
Paradeisstrasse 20 D
D-8120 Weilheim (DE)**

(74) Vertreter : **Weickmann, Heinrich, Dipl.-Ing. et al
Patentanwälte Dipl.-Ing. H. Weickmann Dipl.-Phys.Dr.
K. Fincke Dipl.-Ing. F.A. Weickmann Dipl.-Chem. B.
Huber Dr.-Ing. H. Liska Möhlstrasse 22
D-8000 München 86 (DE)**

Verfahren zur Gewinnung von Cholesterinesterase

Die Erfindung betrifft ein Verfahren zur Gewinnung von Cholesterinesterase aus Mikroorganismen.

Cholesterinesterase spielt eine wichtige Rolle in der klinischen und biochemischen Analytik seit Verfahren zur enzymatischen Bestimmung von Cholesterin entwickelt wurden. Da ein großer Teil des Cholesterins in biologischem Material in Form von Estern vorliegt, ermöglicht die gemeinsame Verwendung von Cholesterinesterase und Cholesterin oxidierenden Enzymen, wie Cholesterinoxidase oder Cholesterindehydrogenase, eine vollenzymatische Bestimmung auch von Cholesterinestern. Dies ist bekannt aus DE-PS 2 264 847. Als besonders geeignet hat sich im Rahmen der Cholesterinesterase-Bestimmung dabei das Enzym aus Mikroorganismen erwiesen (DE-OS 25 06 712.3). Ein Nachteil der bisher aufgefundenen Mikroorganismen mit einem die Aufarbeitung lohnenden Gehalt an Cholesterinesterase besteht jedoch in den relativ geringen Ausbeuten an enzymaktivität, die dabei erhalten werden.

Nunmehr wurde überraschenderweise gefunden, daß bei Verwendung bestimmter Mikroorganismen um ein Vielfaches höhere Aktivitäten erzielt werden können, als dies bisher möglich war. Das erfindungsgemäße Verfahren zur Gewinnung von Cholesterinesterase aus Mikroorganismen der Gattung Pseudomonas ist daher dadurch gekennzeichnet, daß man Pseudomonas sp. DSM 1 280 oder DSM 1 281 in einem geeigneten Nährmedium in Gegenwart eines Induktors züchtet und das Enzym aus der Kulturflüssigkeit oder/und den Zellen gewinnt.

Aus der DE-OS 25 27 068 sind zwar bereits Stämme der Gattung Pseudomonas fluorescens bekannt, welche ebenfalls einen zur Aufarbeitung lohnenden Gehalt an Cholesterinesterase besitzen. Aber auch in diesen Fällen sind die erhältlichen Aktivitätseinheiten gering und liegen vielfach nur bei wenigen Einheiten/Liter. Demgegenüber werden mit den erfindungsgemäß verwendeten Mikroorganismen Aktivitäten von 15 000 und mehr Einheiten/Liter erzielt.

Im Rahmen der erfindungsgemäßen Verfahrens erfolgt die Züchtung in einem Nährmedium, welches einen Induktor enthält. Unter « Induktor » wird hierbei eine Substanz verstanden, welche den Mikroorganismus dazu anregt, das gewünschte Enzym in größerer Menge zu bilden als ohne Induktor. Vorzugsweise wird der eingesetzte Induktor dabei auch als Kohlenstoffquelle, insbesondere als alleinige Kohlenstoffquelle, verwendet. Es ist jedoch auch möglich, gesonderte Kohlenstoffquellen zuzusetzen, beispielsweise Maisquellwasser, Peptone, Hefeextrakte sowie, weniger geeignet, Zucker oder Polyalkohole, wie Glycerin. Als Induktoren erwiesen sich die Palmitinester gut geeignet, insbesondere das Tripalmitin. Die besten Ergebnisse wurden jedoch unter Verwendung von Lecithin als Induktor erzielt. Als besonders geeignet unter den verschiedenen Lecithinen erwies sich Sojalecithin, aber auch andere Lecithinarten, wie Eilecithin oder Hirnlecithin ergaben sehr gute Resultate.

Die eingesetzte Induktormenge ist bis zu einem gewissen Grad abhängig von der Art des verwendeten Induktors. Sie liegt im allgemeinen zwischen etwa 0,1 und 5 Gew.-%, bezogen auf das Volumen des Nährmediums. Bei Verwendung von Lecithin als Induktor und alleinige Kohlenstoffquelle wurden besonders gute Ergebnisse bei einer Menge von 0,5 bis 2 Gew.-% erhalten.

Das Nährmedium enthält darüber hinaus noch üblicherweise zugesetzte Salze und Spurenelemente und sollte durch Zugabe eines geeigneten Puffers auf einen pH-Wert zwischen etwa 5 und 9, vorzugsweise 6 und 8, eingestellt werden. Als Puffer wird Phosphat-Puffer bevorzugt. Darüber hinaus enthält das Nährmedium zweckmäßig noch Ammonium-, Chlor-, Fe-, Cu-, Zn-, Mg- und Ca-Ionen, abgesehen von den Alkaliionen des Phosphatpuffers. Phosphat liegt dabei zweckmäßig in einer Konzentration zwischen 0,4 und 2 Gew.-% vor, jedoch können auch größere oder kleinere Konzentrationen eingesetzt werden.

Tierische Fette und überraschenderweise auch Cholesterinester erwiesen sich im Rahmen des erfindungsgemäßen Verfahrens als Induktoren wenig vorteilhaft.

Ein im Rahmen der Erfindung bevorzugtes Nährmedium besitzt etwa folgende Zusammensetzung, jeweils bezogen auf 1 l Flüssigkeit :

5 bis 10 g, vorzugsweise 6 bis 8 g, $Na_2HPO_4 \cdot 2H_2O$ ;

1 bis 5 g, vorzugsweise 2 bis 4 g, $KH_2PO_4$ ;

0,2 bis 2 , vorzugsweise 0,8 bis 1,2 g, $NH_4Cl$ ;

0,01 bis 0,1, vorzugsweise 0,3 bis 0,7 g, NaCl ;

0,01 bis 1 ml 1 %-ige $FeCl_3$-Lösung ;

0,01 bis 1 ml 0,2 %-ige $CuCl_2$-Lösung ;

0,01 bis 1 ml 1 %-ige Zinksulfat-Lösung ;

0,1 bis 10 ml 10 %-ige $CaCl_2$-Lösung ;

1 bis 20 , vorzugsweise 3 bis 10 ml 12 %-ige $MgSO_4$-Lösung ;

0,1 bis 5 , vorzugsweise 0,5 bis 2 Gew.-% Sojalecithin.

Die Züchtung wird unter aeroben Bedingungen durchgeführt. Es eignen sich sowohl Schüttelkultur als auch belüftete Submerskultur. Die Temperatur kann zwischen etwa 15 und etwa 45 °C liegen, bevorzugt werden 25 bis 35 °C. Maximale Enzymausbeuten erhält man im allgemeinen bereits nach 1- bis 2-tägiger Kulturdauer.

Die erfindungsgemäß verwendeten Mikroorganismen sind sich sehr ähnlich und weisen nachstehende Merkmale auf :

Gram-negativ,
obligat aerob, gelbliche Kolonien auf Fleischpeptonagar,
Wachstum bei 25, 30 und 37 °C,
kein Wachstum bei 10 °C, mehr als 41 °C,
Zellgröße : 0,8 bis 1 μ · 2 bis 4 μ,
motil, lophotrich begeißelt,
neigt zur Kettenbildung,
keine Sporen oder Dauerstadien.

Cytochrom-oxidase +
Katalase (+)
Indol −
Nitrit −
Voges-Proskauer −
Gelatine −

Monosubstrat-Abbau :
Adonit +
Citrat +
Galactose +
Glucose +
Inosit +
Lactose +
Mannit +
Mannose +
Salicin +
Sorbit +

Die Cholesterinesterase tritt sowohl im Kulturmedium als auch in den Zellen auf. Durch Zusatz von oberflächenaktiven Mitteln, insbesondere von nichtionogenen Mitteln, die vorzugsweise dem Typus der Polyoxyäthylenester und -äther mit Alkyl- und Aralkylresten angehören, läßt sich das Verteilungsmuster zwischen Kulturbrühe und Zellen beeinflussen im Sinne einer Erhöhung der extrazellulären Aktivität auf Kosten der intrazellulären Aktivität, bei ionogenen oberflächenaktiven Mitteln verläuft die Änderung der Verteilung in umgekehrter Richtung.

Nach beendeter Züchtung wird die Cholesterinesterase aus der Zellmasse und/oder dem Kulturfiltrat nach üblichen Methoden isoliert und gegebenenfalls gereinigt. Für viele Zwecke eignet sich jedoch bereits ein ungereinigtes Rohprodukt, welches im wesentlichen nur aus aufgeschlossener Zellmasse besteht. Zum Aufschließen eignen sich die dem Fachmann hierfür bekannten Methoden, die hier keiner näheren Erläuterung bedürfen. Sowohl aus dem Kulturfiltrat als auch aus der aufgeschlossenen Zellmasse läßt sich nach Abtrennung von unlöslichen Bestandteilen das Enzym durch Fällung mit üblichen Fällungsmitteln, beispielsweise Salzen, wie Ammoniumsulfat, oder organischen Lösungsmitteln, wie Aceton oder Alkohol, fällen und dann unter Anwendung der üblichen Fraktionierungsmethoden, wie Chromatographie und Fällung, weiter aufreinigen.

Die folgenden Beispiele erläutern die Erfindung weiter.

Beispiel 1

Pseudomonas spec. DSM 1 280, aus der Tiefkühlampulle auf Schrägröhrchen gebracht, wird im Hauptkulturmedium zwei Tage bei 30 °C aerob (Schüttelkolben) vorkultiviert und dann zu 10 % in ein Medium überimpft, welches pro Liter folgende Zusammensetzung aufweist :

| 7 | g $Na_2HPO_4 \cdot 2H_2O$ |
| 3 | g $KH_2PO_4$ |
| 1 | g $NH_4Cl$ |
| 0,05 | g NaCl |
| 0,1 | ml $FeCl_3$, 1 % |
| 0,1 | ml $CuCl_2$, 0,2 % |
| 0,1 | ml $ZnSO_4$, 1 % |
| 1,0 | ml $CaCl_2$, 10 % |
| 5,0 | ml $MgSO_4$, 12 % |
| 1,5 | % Sojalecithin, pH 7,0. |

Die Kultivierung erfolgt bei 30 °C aerob im Schüttelkolben. Nach 1 bis 3 Tagen werden Aktivitäten von etwa 15 000 U/l (Überstand und Biomasse ; Substrat : Cholesteryl-oleat) erhalten.

Etwa gleiche Ausbeuten werden erhalten, wenn unter gleichen Bedingungen anstelle von Pseudomonas spec. DSM 1 280 Pseudomonas spec. DSM 1 281 verwendet wird.

**Ansprüche**

1. Verfahren zur Gewinnung von Cholesterinesterase aus Mikroorganismen der Gattung Pseudomonas, dadurch gekennzeichnet, daß man Pseudomonas sp. DSM 1 280 oder DSM 1 281 in einem geeigneten Nährmedium in Gegenwart eines Induktors züchtet und das Enzym aus der Kulturflüssigkeit oder/und den Zellen gewinnt.

2. Verfahren nach Anspruch 1, dadurch gekennzeichnet, daß man in Gegenwart von Lecithin als Induktor züchtet.

3. Verfahren nach Anspruch 2, dadurch gekennzeichnet, daß man Sojalecithin zusetzt.

4. Verfahren nach Anspruch 2 oder 3, dadurch gekennzeichnet, daß man in Gegenwart von 0,5 bis 2 Gew.-% Lecithin züchtet.

5. Verfahren nach einem der vorhergehenden Ansprüche, dadurch gekennzeichnet, daß man dem Medium 0,4 bis 2 Gew.-% Phosphat zusetzt.

6. Verfahren nach einem der vorhergehenden Ansprüche, dadurch gekennzeichnet, daß man den Induktor als alleinige Kohlenstoffquelle verwendet.

7. Verfahren nach einem der vorhergehenden Ansprüche, dadurch gekennzeichnet, daß man den pH-Wert zwischen 6 und 8 einstellt.

8. Verfahren nach Anspruch 7, dadurch gekennzeichnet, daß man Phosphatpuffer verwendet.

9. Verfahren nach einem der vorhergehenden Ansprüche, dadurch gekennzeichnet, daß man bei einer Temperatur zwischen 25 und 35 °C arbeitet.

10. Verfahren nach einem der vorhergehenden Ansprüche, dadurch gekennzeichnet, daß man zur Erhöhung der extrazellulären Aktivität oberflächenaktive Polyäthylenester und -äther mit Alkyl- und Aralkylresten zusetzt.

## Claims

1. Process for obtaining cholesterol esterase from micro-organisms of the genus Pseudomonas, characterised in that one cultures Pseudomonas sp. DSM 1 280 or DSM 1 281 in a suitable nutrient medium in the presence of an inducer and obtains the enzyme from the culture liquid and/or the cells.

2. Process according to claim 1, characterised in that one cultures in the presence of lecithin as inducer.

3. Process according to claim 2, characterised in that one adds soya lecithin.

4. Process according to claim 2 or 3, characterised in that one cultures in the presence of 0.5 to 2 % by weight of lecithin.

5. Process according to one of the preceding claims, characterised in that one adds 0.4 to 2 % by weight of phosphate to the medium.

6. Process according to one of the preceding claims, characterised in that one uses the inducer as sole source of carbon.

7. Process according to one of the preceding claims, characterised in that one adjusts the pH value to between 6 and 8.

8. Process according to claim 7, characterised in that one uses phosphate buffer.

9. Process according to one of the preceding claims, characterised in that one operates at a temperature between 25 and 35 °C.

10. Process according to one of the preceding claims, characterised in that for increasing the extracellular activity, one adds surface-active polyethylene esters and ethers with alkyl and aralkyl radicals.

## Revendications

1. Procédé de préparation de cholestérol estérase à partir de microorganismes du genre Pseudomonas, caractérisé en ce qu'on cultive Pseudomonas sp. DSM 1 280 ou DSM 1 281 dans un milieu de culture approprié en présence d'un inducteur et en ce qu'on obtient l'enzyme à partir du liquide de culture ou/et des cellules.

2. Procédé suivant la revendication 1, caractérisé en ce qu'on cultive en présence de lécithine comme inducteur.

3. Procédé suivant la revendication 2, caractérisé en ce qu'on ajoute de la lécithine de soja.

4. Procédé suivant la revendication 2 ou 3, caractérisé en ce qu'on cultive en présence de 0,5 à 2 % en poids de lécithine.

5. Procédé suivant l'une des revendications précédentes, caractérisé en ce qu'on ajoute au milieu 0,4 à 2 % en poids de phosphate.

6. Procédé suivant l'une des revendications précédentes, caractérisé en ce qu'on utilise l'inducteur comme source unique de carbone.

7. Procédé suivant l'une des revendications précédentes, caractérisé en ce qu'on ajuste la valeur du pH entre 6 et 8.

8. Procédé suivant la revendication 7, caractérisé en ce qu'on utilise un tampon phosphate.

9. Procédé suivant l'une des revendications précédentes, caractérisé en ce qu'on travaille à une température comprise entre 25 et 35 °C.

10. Procédé suivant l'une des revendications précédentes, caractérisé en ce que pour augmenter l'activité extracellulaire, on ajoute des esters et éthers de polyoxyéthylène avec des radicaux alcoyle et aralcoyle, tensioactifs.